Europäisches Patentamt

European Patent Office

Office européen des brevets

(19)

(11) Publication number: **0 282 057**
**A2**

(12) **EUROPEAN PATENT APPLICATION**

(21) Application number: **88103801.2**

(22) Date of filing: **10.03.88**

(51) Int. Cl.⁴: **A61K 43/00** , A61K 49/00 ,
//(A61K43/00,39:395,31:40),
(A61K43/00,39:395,31:475),
(A61K43/00,39:395,31:70),
(A61K43/00,39:395,31:505),
(A61K43/00,39:395,31:71),
(A61K43/00,39:395,31:19)

(30) Priority: **11.03.87 US 30700**

(43) Date of publication of application:
**14.09.88 Bulletin 88/37**

(84) Designated Contracting States:
**AT BE CH DE FR GB IT LI LU NL SE**

(71) Applicant: **The Regents of the University of Michigan**
**225 West Engineering Building**
**Ann Arbor Michigan 48109(US)**

(72) Inventor: **Sinkule, Joseph A.**
**14302-20th Drive S.E.**
**Mill Creek Washington 98012(US)**
Inventor: **Buchsbaum, Donald J.**
**8800 Pellett Drive**
**Whitmore Lake Michigan 48189(US)**

(74) Representative: **Brown, John David**
**FORRESTER & BOEHMERT**
**Widenmayerstrasse 4/I**
**D-8000 München 22(DE)**

(54) **Chemo-radio-immuno-conjugates.**

(57) Chemo-radio-immunoconjugates (CRIC) and methods of their making are described. The CRIC comprises a complex of chemotherapeutic agent, radionuclide and antibody. The conjugate is suitable for therapeutic uses.

# CHEMO-RADIO-IMMUNO-CONJUGATES

## Background of the Invention

The present invention relates to antibody conjugates useful in the diagnosis and treatment of cancer, AIDS, or other diseases. More particularly, the present invention relates to a conjugate of a chemotherapeutic agent, a radionuclide, and an antibody which can be used to monitor the pharmacokinetics of the conjugate in vivo and for therapeutic effect.

The use of radionuclides and chemical therapeutic agents having a cytotoxic effect has long been known in the treatment of tumors and other pathologic conditions. More recently, antibodies have also been utilized against a wide variety of target antigens, including tumor cells. Monoclonal antibodies in particular offer a high degree of specificity which facilitates the destruction of cancerous cells while minimizing the damage to normal cells.

The combination of antibodies and radionuclides is taught in the patent literature. U.S. Patent No. 4,472,509 September 18, 1984 to Gansow et al. discloses metal chelate conjugated monoclonal antibodies. The chelated metal may be one which emits alpha, beta, or gamma radiation or positrons, and the conjugates are taught to be suited for diagnostic and therapeutic uses. U.S. Patent No. 4,430,318 February 7, 1984 to Langone relates to a method for the preparation of $^{125}$I-labeled protein A of high specific and functional activity. Other patents disclosing radiolabeled antibodies include U.S. Patent Nos. 4,331,647 May 25, 1982; 4,348,376 September 7, 1982; 4,361,544 November 30, 1982; 4,444,744 April 24, 1984; 4,460,559 July 17, 1984; and 4,460,561 July 17, 1984 all to Goldenberg. Further radiolabeled antibodies are disclosed in U.S. Patent No. 4,311,688 January 19, 1982 to Burchiel et al. and 3,927,193 December 16, 1975 to Hansen et al.

Complexes of radionuclides and certain cytotoxic chemical agents are also known in the patent literature. U.S. Patent No. 4,620,971 November 4, 1986 to Hou discloses an indium-bleamycin complex for clinical use as a radiopharmaceutical. U.S. Patent No. 4,485,086 November 27, 1984 to Wong discloses a method of radiolabeling porphyrin compounds with $^{111}$In and $^{113m}$In. The disclosures of all of the above patents are specifically incorporated by reference herein.

Despite the above advances, there remains a need for therapeutic agents exhibiting greater selectivity or specificity for destroying malignant cells, while minimizing the damage to normal tissues. There also remains a need for improved therapeutic agents that are efficacious against cells that have developed radiation resistance but not chemotherapy resistance, and against cells that have developed chemotherapy resistance but not radiation resistance.

## Summary of the Invention

It has now been found that a complex or conjugate of an antibody, radionuclide and chemotherapeutic agent having improved therapeutic efficacy can be made. In accordance with the present invention, a chemo-radio-immuno-conjugate is provided. The radioactive component is used to potentiate the therapeutic effect of the chemotherapeutic agent of the conjugate although it can, in some cases, also be used to monitor the distribution of the conjugate in vivo. Further understanding of this invention will be had from the following disclosure.

A chemo-radio-immuno-conjugate of the present invention comprises a conjugate of a chemotherapeutic agent, a radionuclide, and an antibody. Preferably, the chemotherapeutic agent is selected from the group consisting of antitumor antibiotics, porphyrin and related compounds, vinca alkaloids, antitumor alkylating agents, and folic acid analogs (anti-fols), and the antibody is preferably monoclonal. To maximize the therapeutic effect of the conjugate, the radionuclide is preferably a beta- or alpha-emitter.

## Description of the Invention

The present invention generally relates to a chemo-radio-immuno-conjugate. The "chemo" component is a chemotherapeutic agent. By the term chemotherapeutic agent is meant a low molecular weight, i.e., less than 10,000 MW, chemotherapeutic agent clinically useful against solid tumors, leukemias, viral infections or a variety of malignancies and pathologic states. The chemotherapeutic agent of the present invention is preferably selected from the group consisting of antitumor antibiotics, porphyrin and related compounds, vinca alkaloids, antitumor alkylating agents and anti-fols. More preferably, the chemotherapeutic agent is selected from the group consisting of daunorubicin, doxorubicin, methotrexate, aminopterin, vinblastine, vindesine, bleomycin, blenoxanc, hematoporphyrin derivative, dihematoporphyrin ether, mitamycin, mithramycin and chlorambucil. These and other suitable chemotherapeutic agents are well-known in the art, and are in current clinical use and commercially available.

In alternative embodiments of the invention, other therapeutic agents may be attached to an antibody, and a radionuclide is bound thereto to form therapeutic agent-radio-immunoconjugates. The therapeutic agent chosen for use will vary according to the nature of the disease to be treated, e.g., the type of target cells to be eradicated in vivo. Examples of therapeutic agents which may be used (in addition to the above-described chemotherapeutic drugs) are biologic response modifiers, toxins, and fragments thereof, among others. Various conjugates comprising an antibody, at least one therapeutic agent, and at least one radionuclide are provided by the present invention.

Examples of biologic response modifiers (BRMs) are interferons (alpha, beta, and gamma), tumor necrosis factor, lymphotoxin, and interleukins (IL-1, -2, -3, -4, -5, and -6). Examples of toxins which may be employed are ricin, abrin, diphtheria toxin, Pseudomonas exotoxin A, ribosomal inactivating proteins, and mycotoxins; e.g., trichothecenes. Trichothecenes are a species of mycotoxins produced by soil fungi of the class fungi imperfecti or isolated from Baccharus megapotamica (Bamburg, J.R., Proc. Molec. Subcell Bio. 8:41-110, 1983; Jarvis & Mazzola, Acc. Chem. Res. 15:338-395, 1982).

Therapeutically effective modified toxins or biological response modifiers or fragments thereof, such as those produced through genetic engineering or protein engineering techniques, may be used. Hybrids prepared by joining together two or more toxins or two or more BRMs, or fragments thereof, may also be used.

Radionuclides, the "radio" component, which can be used in the present invention can be any radionuclide suitable for therapeutic use. Radionuclides include gamma-emitters, position-emitters, X-ray emitters or fluorescence-emitters, but, for therapeutic use, are preferably beta-or alpha-emitters. Of course, it may be desirable to employ a nontherapeutic radiolabel for imaging purposes, as well as a therapeutic radionuclide. Broadly speaking, radionuclides are well-known in the art and include $^{123}I$, $^{125}I$, $^{130}I$, $^{131}I$, $^{133}I$, $^{135}I$, $^{47}Sc$, $^{72}As$, $^{72}Se$, $^{90}Y$, $^{88}Y$, $^{97}Ru$, $^{100}Pd$, $^{101m}Rh$, $^{119}Sb$, $^{128}Ba$, $^{197}Hg$, $^{211}At$, $^{212}Bi$, $^{212}Pb$, $^{109}Pd$, $^{111}In$, $^{67}Ga$, $^{68}Ga$, $^{67}Cu$, $^{75}Br$, $^{77}Br$, $^{99m}Tc$, $^{11}C$, $^{13}N$, $^{15}O$ and $^{18}F$. Those radionuclides suitable for therapeutic use are well-known in the art. Preferred radionuclides are $^{125}I$, $^{131}I$, $^{90}Y$, $^{67}Cu$, $^{188}Re$, $^{186}Re$, $^{211}At$ and $^{212}Bi$.

For the "immuno" component of the present invention, generally any antibody may be utilized, including polyclonal and monoclonal antibodies.

However, monoclonal antibodies are preferable because, in contrast to the heterogenous mixtures of immunoglobulins composing polyclonals, monoclonal antibodies have a well-defined homogenous structure. Due to this homogeneity, monoclonal antibodies exhibit a high degree of reproducibility of function and specificity. Methods for obtaining monoclonal antibodies have been extensively discussed and are well-known in the art. Monoclonal antibodies have already been developed for a wide variety of target antigens, including tumor cells. The selection of monoclonal antibody for the practice of this invention will depend upon the end use for which the conjugate will be employed and such selection is within the skill of the art.

It should be appreciated that, although for simplicity of discussion the description discusses the conjugate of the invention as comprising a single chemotherapeutic agent, radionuclide and antibody, more than one of each of these components can be incorporated into the conjugate. For example, the conjugate may comprise more than one type of radionuclide. Also, the conjugate may comprise more than one therapeutic agent. In one embodiment of the invention, a chemotherapeutic drug, a therapeutic toxin, and a radionuclide may all be attached to an antibody molecule to form a conjugate. Other embodiments of the invention are directed to conjugates comprising various combinations of diagnostic radionuclides, therapeutic radionuclides, toxins or therapeutically effective fragments thereof, biological response modifiers or therapeutically effective fragments thereof, and chemotherapeutic agents, wherein the conjugate comprises an antibody, at least one therapeutic agent, and at least one radionuclide.

The therapeutic/cytotoxic action of the conjugate can be achieved by a whole host of mechanisms. The mechanisms of action of the conjugate will of course depend upon which components are selected to form the conjugate and their respective mechanisms of action, as well as upon the specific target on which the conjugate acts. The mechanisms of action of chemotherapeutic agents, for example, include enzyme inhibition or other protein binding or an effect on nucleic acids which can lead to cell death or prevent cell proliferation. Ionizing radiation from the radionuclide of the conjugate can, for example, affect the DNA and/or RNA and/or the membrane of the cell, also preventing cell proliferation, and potentiating the effect of the chemotherapeutic agent.

The therapeutic effects of the therapeutic agent and the radionuclide may be synergistic, rather than just additive, in some cases. The mechanisms for the synergistic effect may vary, according to the combination of therapeutic agent and radionuclide present on a particular immunoconjugate of the invention. For example, certain target cells able to survive the cytotoxic effect of one of the therapeutic moieties, e.g., the "chemo" or the "radio" moiety, may be sufficiently weakened to succumb to the effects of the second therapeutic moiety. In addition, tumors often comprise heterogeneous populations of cancer cells, some of which may be more effectively eradicated by one of the effector moieties than the other. Thus, delivery of both types of effectors may increase the chances of destroying different populations of cancer cells within a tumor.

The therapeutic activity of the conjugate in vivo is localized by the antibody, which is selected for its specificity for the target cell or biomaterial. Thus, the chemo-radio-immuno-conjugates of the present invention can be used in the treatment of any pathologic condition in which the destruction of a target area or biomaterial is desirable for therapy, as long as an antibody to the target can be produced.

Alternatively, targeting proteins other than antibodies may be used to deliver the therapeutic agents and the radionuclides to a desired target site within a human or mammalian host. The term "targeting protein" as used herein refers to proteins which are capable of binding to a desired target site in vivo. The targeting protein may bind to a receptor, substrate, antigenic determinant, or other binding site on a target cell or other target site. Examples of targeting proteins include, but are not limited to, antibodies and antibody fragments, certain serum proteins and hormones. These proteins may be modified, e.g., to produce variants and fragments of the proteins, as long as the desired biological property (i.e., the ability to bind to the target site) is retained. The proteins may be modified by using various genetic engineering or protein engineering techniques.

The target site may comprise target cells to be eradicated. In one embodiment of the invention, the target cells to be eradicated are cancer cells. A number of monoclonal antibodies and fragments thereof specific for different cancer cell-associated antigens have been developed. The choice of antibody will depend on the type of cancer with which the patient is afflicted.

The therapeutic effects of the conjugate can be exerted upon the target in various ways. For example, the conjugate may remain on the surface of a target cell, release the chemotherapeutic agent in a proactive state which is taken up by the cell. Alternatively, the intact conjugate may be modulated and enter into the cell through endocytosis, whereafter the conjugate is cleaved and the agent exerts its effect. The conjugate may, of course, also be toxic without entering the cell. The radionuclide of the conjugate, whether within or without the cell, will also exert its irradiating effect to potentiate the therapeutic effect of the conjugate.

The various conjugates of the present invention may additionally comprise a diagnostically effective radionuclide. An advantage of using such conjugates is that biodistribution of the conjugate in vivo can be monitored. A number of diagnostically effective radionuclides which are detectable by external means may be used, such as $^{99m}$Tc, $^{123}$I, and $^{111}$In, among others. The biodistribution of these radionuclides may be detected by scanning the patient with a gamma camera after allowing a suitable length of time for localization of the conjugate at the target site. Certain therapeutically effective radionuclides may also function as diagnostically effective radionuclides. Two such radionuclides are $^{186}$Re and $^{188}$Re which have therapeutic use, but also are detectable by gamma camera.

The present invention thus provides a method of monitoring biodistribution of a therapeutically effective conjugate in vivo, comprising the steps of:

a) attaching a diagnostically effective radionuclide to a conjugate comprising an antibody, a therapeutic agent, and a therapeutically effective radionuclide;

b) administering the resulting conjugate comprising the diagnostically effective radionuclide to a human or mammalian host; and

c) detecting the biodistribution of the diagnostically effective radionuclide within the host.

Monitoring the biodistribution of the administered conjugate allows the physician to determine whether the conjugate has localized at all target sites (including metastases) in vivo (the target sites having been identified previously during a diagnostic procedure). The ability to monitor biodistribution may be especially important when certain therapeutic agents (e.g., relatively large agents such as protein toxins) are used, to ensure that attachment of the therapeutic agent to the targeting protein has not altered the ability of the targeting protein to bind to the target sites.

The present invention can be used, for example, in the treatment of tumors, AIDS, and viral infections. In the treatment of such conditions, the conjugates of the present invention are employed in therapeutically safe and effective amounts, i.e., amounts which achieve the desired clinical result without undue toxicity. The conjugates can be administered by any conventional procedure, topically, orally, intravenously, parenterally, and the like. In addition to chemo-radio-immuno-conjugate, pharmaceutical compositions can further contain components such as pharmaceutically-acceptable carrier, excipients, additives or other conjugates of the present invention. Appropriate concentrations of bioactive therapeutic materials, such as those contemplated by the present invention, in such pharmaceutical compositions will depend upon several factors and can be routinely determined by those skilled in the art. The effective dose of a chemo-radio-immuno-conjugate of the present invention in any application will be dependent upon the particulars of that application. In treating tumors, for example, the dose will depend, inter alia, upon tumor burden, accessibility and other variables.

## Method of Making Chemo-Radio-Immuno-Conjugates

It is important that the conjugates of the present invention be made by a method which: (a) retains therapeutic activity; (b) retains antibody activity and specificity; (c) allows sufficient molar incorporation ratio to deliver toxic amounts of chemotherapeutic agent and radionuclide to the target; (d) minimizes polymerization or aggregation of conjugates; and (e) is technically reproducible. Since the conjugate must be delivered to the target cells in a form that is bioactive, care must be taken to avoid affecting reactive groups of the components which could adversely alter the conjugate's biological activity. For example, the chloroethylamine groups on alkylating agents such as L-phenylalanine mustard, chlorambucil or uracil mustard and the chloride leaving groups on cisplatin must be protected during synthesis to obtain active conjugates. Similarly, the active portion of the anti-fol, methotrexate (MTX), must not be modified so as to preserve binding to the target enzyme dihydrofolate reductase.

There are a number of reactive functional groups available for linkage to the antibody. Reactive groups available on the amino acids of antibodies include: (a) the C-terminal carboxyl groups of aspartic and glutamic acid residues; (b) the guanidino group of arginine; (c) the imidazole function of histidine; (d) the phenolic hydroxyl group on tyrosine; (e) the amino groups of the N-terminal and lysine residues; (f) the sulfhydryl groups of cysteine and the thioether of methionine. Hydroxyl groups on serine or carbohydrate prosthetic groups furnish additional sites for linkage.

The selection of a method for linkage of a chemotherapeutic agent, radionuclide and antibody is generally dependent upon the type of bond required (e.g., covalent or non-covalent) and the reactive moieties involved. Reactive groups already extant on the components of the conjugate may be used for direct linkage of these components. Alternatively, reactive moieties may be introduced to one or more of the conjugate components to provide a linking mechanism. For example, spacers may be introduced to provide a more favorable steric orientation of the chemotherapeutic agent and/or radionuclide relative to the conjugate. Intermediates which can incorporate and/or bind the components of the conjugate while preserving the bioactivity of the conjugates may also be used. Bonding may also be achieved through non-covalent interactions of the components.

Thus, methods of linkage between a chemotherapeutic agent, a radionuclide and an antibody may be generally grouped as: (1) direct, covalent coupling, which may require modification of one or more of the components of the conjugate; (2) indirect binding of the chemotherapeutic agent and/or radionuclide to the antibody through a spacer or bridge, or in the case of radiometals, through a chelator; (3) coupling of the components through degradable or nondegradable intermediates such as the dextrans, poly-glutamic and poly-lysine, or serum albumin or liposomes; and (4) non-covalent bonding, e.g., through ionic or hydrophobic interaction.

Illustrative of the first method, direct linkage of a chemotherapeutic agent to an antibody can be achieved by initial modification of either the antibody or the agent, followed by direct interaction and covalent binding to the modified groups. For example, the hydrazinolysis of chemotherapeutic agent vinblastine (Velban™) yields deacetylvinblastine hydrazide. This compound can then be converted into the acid azide by the action of nitrous acid and the acid azide group can then react with nucleophilic sites on an antibody such as amino and sulfhydryl groups on immunoglobulin G (IgG). Four to eight molecules of vinblastine can be coupled per IgG molecule with this approach. Alternatively, the N-hydroxy succinimide ester of vinblastine can be synthesized and coupled to amino groups on KSI/4, an IgG2a that recognizes a 40 k dalton epithelial antigen found on human lung, breast and colon adenocarcinomas.

Another example of direct linkage is the linkage between methotrexate and antibody through the treatment of methotrexate with acetic anhydride to form mixed anhydride, whereafter the resultant product is added directly to antibody.

Another form of direct linkage between certain chemotherapeutic agents and antibodies include chemical conjugation between a carbohydrate group on the antibody and an amino group on the chemotherapeutic agent or vice versa, through periodate oxidation of vicinal hydroxyl on the carbohydrate moiety. Chemotherapeutic agents amenable to this method of linkage include anthracycline antibiotics such as doxorubicin and daunorubicin, which contain a daunosamine sugar. Toxic ribonucleosides or ribonucleoside-analogs containing adjacent hydroxyl groups can also be directly coupled to antibodies using mild, aqueous reaction conditions. For example, the 2'-and 3'-

hydroxyls on the ribose sugars of chemotherapeutic agents such as cytosine arabinoside, fluorodeoxyuridine or other nucleoside analogs would be amenable towards this type of linkage. Alternatively, the 5'-hydroxyl groups could be catalytically oxidized to the corresponding 5'-carboxylic acid and then coupled as per a carboxylic acid linkage. The hydroxyls on ribose sugar-containing analogs can be periodate oxidized to aldehydes and coupled directly to amino-containing proteins such as antibodies.

Similarly, carbohydrate functions on antibody immunoglobulins can be modified for site-specific coupling of amino-containing chemotherapeutic agents. It is well-known that antibodies contain variable amounts of carbohydrates, for example, mannose, N-acetylglucosamine, galactose, fucose and sialic acid. The carbohydrate portions of the antibody are usually located in the $F_c$portion of the immunoglobulin and rarely in the variable region, thus permitting site-specific modification of immunoglobulin carbohydrates without disrupting antigen binding. Periodate oxidation of adjacent hydroxyls (diols) on antibody carbohydrate moieties generates an aldehyde which is reactive with amino and sulfhydryl functional groups. Other alternative chemical methods for activating carbohydrate groups for linkage are: (1) converting monosaccharides and reducing oligosaccharides to aldonic acids by $Br_2$ or $I_2$ oxidation followed by direct coupling; (2) linking glycosides that have a p-nitro or p-aminophenyl aglycone to proteins by diazotization; or (3) cyanuric chloride may be used and carboxyl or sulfhydryl groups may be introduced.

It will be appreciated that modifications, such as those discussed above, of groups on the components of the conjugate to yield reactive sites for linkage, can be used not only to directly link the components, but to introduce new groups (including intermediates and linkers discussed below) onto the reactive sites which, in turn, can be used to link the components. For example, the aldehyde groups formed by oxidation of hydroxyl functions of carbohydrate group of the antibody can be used to introduce new groups on the antibody for linkage. As a further example, cyanuric chloride (trichloro-S-triazine) or dichloro-S-triazine substituted with carboxyl methylamine groups can be used to couple hydroxyl-containing chemotherapeutic agents to antibodies in a two-step reaction. An ether is formed upon reaction of the hydroxyl with cyanuric acid and, since the hydroxyl is not sufficiently mucleophilic and does not readily react with the chlorides, the second step is to couple the amino groups on the antibody to the triazine moiety on the modified agent.

The radionuclide can also be directly linked to the chemotherapeutic agent or to the antibody. For example, radioisotopes of iodine, such as $^{125}$I or $^{131}$I can be linked to the chemotherapeutic agent or antibody by the monochloride micro method described by Contreras et al. in "Iodine Monochloride (ICl) Iodination Techniques," Methods in Enzymology 92:277-292, 1983.

Illustrative of the second method, chemotherapeutic agents can be coupled to antibodies through spacers or bridges of varied but defined length and composition. Chemotherapeutic agents amenable to this method of linkage include methotrexate, chlorambucil, bleomycin, and mitamycin, as well as anthracycline derivatives. Using this method, cross-linking reagents, for example bifunctional cross-linkers p-benzoquinone, bisoxirane, and gluteraldehyde, can be employed to form bridges and/or spacers between reactive sites (e.g., amino or sulfhydryl group) on the antibody and the chemotherapeutic agent. This method is particularly useful in overcoming steric hindrance between these components by "bridging" the agent a fixed distance off the protein surface to provide a better orientation of the agent. Certain bifunctional reagents, such as bisoxirane, also permit simultaneous introduction of a spacer group in the coupling process and are thus useful when additional spacing between bound components of the conjugate is desired.

In addition to the types of bridges and spacers discussed above, a chelating bridging mechanism can also be employed to bind a radiometal to the conjugate. Generally speaking, a metal chelator or ligand, which binds the radioactive metal to the conjugate, is linked to the antibody. See U.S. Patent No. 4,472,509 September 18, 1984 to Gansow et al. and U.S. Patent No. 4,485,086 November 27, 1984 to Wong. For example, the chemo-radio-immuno-conjugates can be made with a variety of beta-emitting metallic radionuclides using the bicyclic anhydride of DTPA as the chelating agent. See Hnatowich, D.J. et al., in "Radioactive Labeling of Antibody: A Simple and Efficient Method," Science 220:613-615 (1983) and Hnatowich, D.J. et al. "The Preparation of DTPA Coupled Antibodies Radiolabeled with Metallic Radionuclides: An Improved Method." J. Immunol. Methods 65:147-157 (1983). Diamide dimercaptide chelating compounds useful for binding radionuclides such as $^{99m}$Tc, $^{186}$Re and $^{188}$Re to antibodies are described in European Patent Application Publication No. 188,256. The choice of the chelator or ligand will, of course, depend upon the metal to be chelated, and the chelated metal can be any radioactive metal emitting alpha, beta or gamma radiation or positrons, or alternatively a fluorogenic or paramagnetic metal. It should also be appreciated that certain chelators, such as porphyrins, bleomycins, and related compounds, themselves function as chemotherapeutic agents and may thus also comprise the "chemo" component of the conjugate. For example, the chemotherapeutic agents bleomycin, hematoporphyrin derivative, and dihematoporphyrin ether may be used as ligands for the relatively stable attachment of certain radionuclide metals (e.g., $^{67}$Cu, $^{90}$Y, and $^{111}$In) to an antibody.

The third conjugation method, binding through an intermediate, generally involves attaching several molecules of chemotherapeutic agent and/or radionuclide to relatively inert polymeric intermediates, and then coupling the agent intermediate complex to the antibody. The advantages of this conjugation mechanism are two-fold: first, the amount of chemotherapeutic agent per conjugate and the corresponding cytotoxicity of the conjugate is increased, and second, the bioactivity of the conjugate is preserved. For example, methotrexate (MTX) antihuman melanoma (ZME018) conjugates formed using human serum albumin (HSA) intermediates have recently been shown to have improved specificity and a substantial increase in cytotoxicity compared to direct linkage or spacer (EDCI) mediated linkage. It should be noted that the use of an intermediate for conjugation can also be used in conjunction with the bridging and spacer mechanisms discussed above.

Intermediates useful in the present invention include polysaccharides such as the dextrans, poly-glutamic acid and poly-lysine, serum albumin and other polymers such as microspheres or liposomes. For the most part, the preparation of cytotoxic agent-linked intermediates and their coupling to antibody molecules involves many of the same chemistries discussed above. For example, chemotherapeutic agents can be linked to dextrans by direct esterification of dextran hydroxyls or by activation of the hydroxyls by periodate, cyanogen bromide, hydrazides, organic cyanates of halopropyl epoxides. Periodate oxidation and cyanogen bromide activation are the most widely used methods for coupling chemotherapeutic agents to dextrans. Among the procedures which may be used for linking therapeutic agents to polymeric intermediates, which in turn are bound to antibodies, are those described in U.S. Patent Nos. 4,699,794 and 4,046,722.

Finally, the components of the conjugate may be linked through non-covalent bonds such as by ionic interaction or hydrophobic adsorption of the chemotherapeutic agent or radionuclide onto the antibody. For example, porphorins can be bound to antibody through ionic interaction.

Also provided by the present invention is a method for attaching an agent to a protein, comprising the steps of:

a) reacting the protein with a molar excess of the agent under a first set of reaction conditions such that the agent reacts with a first functional group on the protein to bind a first portion of the agent molecules to the protein; and

b) changing the reaction conditions such that unbound agent molecules react with a second functional group on the protein, thereby binding an additional second portion of the agent molecules to the protein.

An advantage of using this method is that more molecules of the agent may be attached to each protein molecule, compared to linking methods that target only one functional group on the protein. The agent may be any of the diagnostic or therapeutic agents described above, including, but not limited to, drugs and bifunctional chelating compounds. The step of changing the reaction conditions may involve any alteration of the conditions in the reaction mixture which causes the agent to react with a different functional group on the protein. Methods of changing the reaction conditions include, but are not limited to, altering the pH or temperature of the reaction mixture, or adding a bifunctional linker molecule reactive with both the agent and the second functional group on the protein. The change in the reaction conditions to be effected will depend on such factors as the chemical structure of the agent. The group on the agent that reacts with the protein may be the same or different in the first and second steps, and may react with the protein directly or through a linker.

One embodiment of this method of the invention is illustrated in Example 10 below. In the first step, doxorubian is bound to an antibody using a water soluble carbodiimide linker at a pH of about 5.5 to 6.0, and about a 100-fold or more excess of doxorubicin. The drug is attached (through the carbodiimide linker) to -COOH groups on the antibody. The pH of the reaction mixture is then raised to about 8.0 to 8.5, and glutaraldehyde, a cross-linking reagent, is added. The unbound drug in the reaction mixture (derivatized by glutaraldehyde) reacts with free amine groups on the lysine residues of the antibody, thereby binding an additional portion of the drug to the antibody.

A further understanding of the present invention may be had from the following examples. In these examples, the antineoplastic drugs, i.e., chemotherapeutic agents, are obtained from commercial sources as pharmaceutical-grade, vialed products, except where otherwise noted. Reagents and chemicals are obtained from Sigma Chemical Co., (St. Louis, Mo.), PD-10 (Sephadex-G-25) columns are purchased from Pharmacia (Uppsala, Sweden) and semi-preparative gel filtration, ion exchange and hydroxylapatite HPLC columns are obtained from Beckman, Waters/Millipore and Biorad, respectively.

## EXAMPLES

### Example 2

A doxorubicin (DOX) immunoconjugate for radionuclide attachment is produced by two different methods: a 5-carbon spacer between the free amine on the daunosamine sugar of DOX with amino and sulfhydryl groups on the antibody and an acid-sensitive cis-aconitic anhydride (cis-AA) and carbodiimide linker which would allow release of DOX intracellularly after digestion in the lysosomes. Doxorubicin (DOX) (100 mM final concentration) is then coupled to 443A6, an antibody (IgG₁) against pulmonary adenocarcinoma (10 mM final reaction concentration in pH 8.5 PBS). See Lee et al., Cancer Research 45:5813 (1985). Dilute glutaraldehyde (0.125%) is added dropwise (100 ul to 900 ul DOX and antibody in pH 8.5 PBS) over 2 minutes at room temperature in the dark. The reaction mixture is placed over a Sephadex-G-25 (PD-10) column to isolate the conjugate from the reactants and the conjugate fraction is sterile-filtered and quantitated. The conjugate fraction is analyzed by uv/vis spectroscopic assays and protein assays to calculate the molar incorporation of DOX onto the antibody. The acid-sensitive linker, cis-AA is first coupled to DOX by adding 5 mg cis-AA to a stirred solution of DOX (5 mg in 1 ml distilled water) adjusted to pH 9 with NaOH. After 10 minutes at room temperature, the solution is further diluted with 2 ml water and placed on ice. Hydrochloric acid (HCl) (0.5N) is added to the cold, stirred solution until a heavy precipitate is formed. The precipitate is isolated, dissolved in 4 ml distilled water and reacidified/precipitated with HCl. The final precipitate is dissolved in 0.5 ml distilled water and pH adjusted to 8.0 with sodium hydroxide (NaOH). The antibody (10 mg in 0.5 ml pH 8 PBS) is added to the DOX-aconitic anhydride solution and 20 mg of 1-ethyl-3-(3-dimethylaminopropyl) carbodiimide (EDCI) is slowly added with stirring. The mixture is incubated for about 2 hours at pH 7.0 and incorporation is monitored by gel filtration HPLC at 280 nm and 475 nm detector wavelengths. The mixture is passed through a PD-10 column and the conjugate fraction is collected. The incorporation ratio is quantitated after sterile filtration and concentration.

### Example 2

Chlorambucil (CBL) immunoconjugates for radionuclide attachment are prepared by covalent linkage of CBL's carboxyl group to amines of antibody of Example 1 by (1) water-soluble 1-ethyl-3-(3-dimethylaminopropyl) carbodiimide (EDCI), or (2) by formation of the mixed anhydride of CBL at low temperature and alkaline pH. In the first method, CBL (62 mg in 2 ml methanol) is treated with 12 mg of sodium methoxide to form the sodium salt of CBL. The methanol is removed and 3 mg of the salt is dissolved in 1.5 ml of PBS containing 15 mg of antibody and EDCI is added (3 mg). The mixture is stirred for 2 hours at 4°C and purified on Sephadex G-25 (PD-10) column. In the second method, the mixed anhydride of CBL is formed at 4°C by first reacting CBL with butyl chloroformate (1/1 molar ratio) in triethylamine. Antibody in pH 9 PBS is added dropwise to the stirred, unpurified mixture at 4°C (anhydride/antibody molar ratio of 50/1) and incubated for 1-2 hours. After high-speed centrifugation (10,000 xg) the supernatant is purified on a PD-10 column and the eluant checked by HPLC and nitrobenzyl pyridine (NBP) alkylation assay.

### Example 3

Methotrexate (MTX) is coupled to antibody of Example 1 via the gamma-carboxyl group of MTX with retention of biological activity. The MTX (0.1 mmol in 1 ml dry dimethyl formamide (DMF)), N-hydroxysuccinamide (0.1 mmol in 0.5 ml dry DMF) and EDCI (0.1 mmol in 0.5 ml DMF) are added in sequence into an amber reaction vial. The mixture is stirred at room temperature for 1 hour, then placed in the refrigerator (4°C) for 20 hours. The mixture is centrifuged (10,000 xg) and the orange supernatant removed, quantitated and stored under N₂ at 80°C until ready for conjugation. The active ester of MTX (0.2 ml) is then added to a stirred solution of antibody (20 mg in 4 ml pH 7 PBS and 2 ml DMF). The components are incubated for 4 hours at 4°C and the mixture is then centrifuged (10,000 mg) for 20 minutes. The supernatant is placed on a PD-10 column and the MTX-antibody conjugate collected and analyzed.

### Example 4

Doxorubicin (DOX) radio-immuno-conjugates are prepared by adding DOX (5-10 mg in 1 ml water) to MB-1, a murine monoclonal antibody (IgG₁ subclass) developed by Link et al. (J. Immunology 137:3013, 1986), in a 5 ml reaction vial containing a magnetic stir bar. The antibody is previously dialyzed against 4 x 4 liter pH 7 PBS exchanges, concentrated to approximately 20 mg/ml and 20 mg is added to 5-10 mg DOX with stirring at 25°C. After 15 minutes, 10 mg of 1-ethyl-3-(3-dimethylaminopropyl) carbodiimide (EDCI) or 8 mg 1-cyclohexyl-3-(2-morpholinoethyl)-

carbodiimide (CMC) metho-p-toluene sulfonate is added and stirred in the dark for 30 minutes at 25°C. A solution of fresh 0.1% glutaraldehyde is added (100 ul/ml reaction volume) dropwise over 60 seconds with rapid stirring. The mixture is incubated another 4-5 minutes, then the whole reaction solution placed over a Sephadex G-25 column (PD-10) which was previously equilibrated in pH 7.8 borate buffered saline (BBS). The DOX/MB-1 conjugate is collected in the third-seventh ml fractions (void volume) off the column. An 10-20 ul aliquot of the 4 ml fraction (containing approximately 15 mg MG-1 and 0.5 mg DOX per ml) is added to an acid-washed 5 ml reaction vial and NaI-125, NaI-123, or NaI-131 (low or high specific activity) is added (the initial 10-20 ul volume of the aliquot is increased to 100 ul with PBS and the whole 100 ul is added to the reaction vial). Iodine monochloride (ICl) was prepared by the method of Contreras et al. (Methods in Enzymology 92:277, 1983) and 5 equivalents were used for MB-1 as for most other IgGs (whereas 10-12 equivalents are used for TgMs). Approximately 60 seconds later, human serum albumin (HSA) (2% final concentration) is added as a protective protein and the chemo-radio-immuno-conjugate is purified over a PD-10 column equilibrated with pH 7.4 PbS and the third-seventh ml collected. Alternatively, the conjugate is purified over a Dowex (1 x 4, 150 mesh, 5 ml column bed, BioRad) column to remove free, unconjugated radioiodine.

## Example 5

A radiometal-porphyrin antibody conjugate is prepared by first adding $^{111}$InCl$_3$ or $^{90}$YCl$_3$ (5-10 mCi) in 0.05 N HCl to 1 ml of pH 4.0 sodium acetate buffer and stirring in an acid-washed amber reaction vial for 10 minutes. Hematoporphyrin derivative (HPD) and/or dihematoporphyrin ether (DHE) (10 mg) is added and the solution is stirred and heated to 80°C on a hot plate for 1-24 hours. The solution is cooled, adjusted to pH 6.0 with 1 N NaOH and 20 mg EDCI or 15 mg CMC is added to derivatize the free carboxyl groups of HPD or DHE. MB-1 antibody (40-50 mg in pH 7.4 PBS) is added dropwise to the stirred solution over 1 minute and incubated 30 minutes further. The derivatized carboxyl groups of the radiometal-porphyrin are attached to amino and hydroxyl groups of the antibody by EDCI. The radiometal-porphyrin/antibody conjugate is purified over a pH 7.4 PBS equilibrated Sephadex G-25 column (PD-10) and the 4 ml fraction containing maximal absorbance at 505 nm, radioactivity and immunoglobulin protein is collected (by HPLC and native PAGE). The product is stable in sterile PBS pH 7 for up to two weeks.

## Example 6

Bleomycin antibody conjugate is prepared as follows: bleomycin (BLEO) (10 units or mg Sigma-grade BLEO) dissolved in pH 4.0 acetate buffer and Y-90 (5 mCi/100 ul 0.05 N HCl, Oak Ridge, Tenn.) are added to an acid washed reaction vial with stirring at 25°C and incubated for 1-2 hours. MB-1 antibody (20 mg in 1 ml pH 8.0 PBS) is added, followed by 10 mg of EDCI or CMC, and the reaction mixture stirred another 20 minutes at 25°C. The reaction vial is centrifuged (3000 RPM for 20 min.) and the supernatant placed over a Sephadex G-25 (PD-10) column and purified.

## Example 7

Doxorubicin is attached to a monoclonal antibody designated NR-LU-10, which binds to a 37-40 kilodalton pancarcinoma glycoprotein, and the resulting drug-antibody conjugate is then radiolabeled. The antibody is previously dialyzed against 4 x 4 liter pH 7 PBS exchanges, concentrated to approximately 20 mg/ml and 20 mg is added to 5 mg DOX (5 mg/ml in water) in a 5 ml reaction vial containing a magnetic stir bar with stirring at 25°C. After 15 minutes, 10 mg of 1-ethyl-3-(3-dimethylaminopropyl)carbodiimide (EDCI) or 8 mg 1-cyclohexyl-3-(2-morpholinoethyl)carbodiimide (CMC) metho-p-toluene sulfonate is added and stirred in the dark for 60 minutes at 37°C. The pH of the reaction mixture is then adjusted to from about 8.0 to 8.5 by adding buffered IN NaOH, pH 8.5, or another suitable buffered solution at pH 8.5. A solution of fresh 0.1% glutaraldehyde is added (100 ul/ml reaction volume) dropwise over 60 seconds with rapid stirring. The mixture is incubated another 4-5 minutes, then the whole reaction solution placed over a Sephadex G-25 column (PD-10) which was previously equilibrated in pH 8.5 phosphate buffered saline (PBS). The DOX/NR-LU-10 conjugate is collected in the third-seventh ml fractions (void volume) off the column, and dialyzed into sodium bicarbonate buffer, pH 9.5.

The drug-antibody conjugate is radiolabeled with $^{211}$At, $^{125}$I or $^{131}$I using the radiohalogenated molecules and procedures described in European Patent Application Publication Number 203,764. For example, the radiolabeling procedure may involve the use of a para-iodo-phenyl compound comprising a short-chain substituent bearing a functional group reactive with an antibody. The functional group may be an ester group which reacts with a free amine group on the antibody to bind the radiohalogenated compound thereto. The radiohalogenated molecule may be prepared by substituting the organo metallic group Sn(n-Bu)$_3$ or

SnMe₃ on a haloaromatic compound which comprises the short chain-functional group substituent. A radioisotope of a halogen then is substituted for the organo-metallic group by halodemetalization.

Alternatively, the drug-antibody conjugate is radiolabeled with $^{99m}$Tc, $^{188}$Re, or $^{186}$Re using the "N₂S₂" chelating compounds and procedures described in European Patent Application Publication Number 188,256. Basically, the radionuclides in the form of pertechnetate or perrhenate are contacted with the chelating compound in the presence of a reducing agent such as stannous ion. The resulting radionuclide metal chelates are attached to the antibody by reacting a functional group on the chelate compound (e.g. an active ester) with a functional group on the antibody (e.g. a free amino group).

Example 8

A chemo-radio-toxin-immunoconjugate is prepared as follows. Pseudomonas exotoxin A (PE) is conjugated to a monoclonal antibody designated NR-ML-05 through a thioether linkage. This monoclonal antibody binds to a 250 kilodalton human melanoma associated proteoglycan.

The PE is first reacted with succinimidyl 4-(N-maleimidomethyl)cyclohexane-1-carboxylate (SMCC) at a molar ratio of 1:10 (protein:linker). Excess heterobifunctional reagent is removed from derivatized PE by gel filtration. NR-ML-05 is treated with 25 mM dithiothreitol (DTT) in 0.01 M phosphate-buffered saline, pH 7.5 (PBS) and excess DTT is removed by gel filtration. The derivatized toxin and the reduced antibody components are mixed and incubated at room temperature for 15⁺ minutes. The conjugation reaction mixture is then fractionated by FPLC gel filtration on a TSK 3000 column at 0.5 ml/min to separate conjugate from unconjugated antibody and unreacted derivatized PE.

The PE/NR-ML-05 conjugate is reacted with doxorubicin according to the procedure described in Example 4 (i.e., reaction with EDCI followed by glutaraldehyde). The same procedure is used in two control reactions to produce a doxorubicin-PE conjugate and a doxorubicin-NR-ML-05 conjugate. The number of doxorubicin molecules attached to PE was 3; attached to NR-ML-05 was 10; while the number of drug molecules attached to PE/NR-ML-05 equals 20.

A radionuclide is attached to the PE/NR-ML-05/doxorubicin conjugate to produce the chemo-radio-toxin-immunoconjugate. One of the radiolabeling procedures described in Examples 4 or 7 above may be used.

Example 9

Four drugs are each attached to a monoclonal antibody (MAb) designated NR-ML-05 in separate reaction mixtures. NR-ML-05 binds to a 250 kd human melanoma associated proteoglycan. The drugs are the anti-cancer drugs doxorubicin, actinomycin D, bisantrene, and mitoxantrone.

Reagent solutions are prepared: a concentrated antibody solution (>5 mg/ml in PBS), concentrated drug (10 mg/ml in dH₂O) and a water-soluble carbodiimide (e.g. EDCI) at 10 mg/ml in dH₂O. Four mg (800 ul) of MAb is first reacted with 2 mg (200 ul) drug for 15 minutes at 37°C in a water bath. Two mg (200 ul) of carbodiimide is added and stirred for 30 minutes at ‾pH 5.5-6.0. Borate buffer (pH 9) is added (700 ul) and pH should be adjusted to pH 8.5 (>pH 9 = purple color). Dilute (0.25%) glutaraldehyde is added (100 ul per 2 mls reaction volume and 4 mg protein) to bring the final volume to 2 mls. This is allowed to react with the drug/antibody until the solution begins to cloud such that one can no longer see through it (approximately 15 minutes). The 2-ml reaction mixture is placed over a PBS-equilibrated Pharmacia PD-10 column or HPLC/GPC column and the 4 ml fraction containing the drug/antibody conjugate is collected. If extensive precipitation occurs, a centrifugation step (3000 rpms at 4°C for 20 minutes) is required. The supernatant is placed over the PD-10 column and the protein content (BioRad method vs. gamma globulin standard at 595 nm) and drug concentration (vs free drug at 3 concentration standards by Vis at 495 nm). If necessary, the product can be concentrated and purified by Ultrafiltration or chromatographically.

The number of drug molecules per antibody molecule, i.e., the "molar incorporation ratio" (MIR), was measured for the NR-ML-05-doxorubicin conjugate by scanning Vis spectra obtained at 400, 450, 475, 495 and 550 nm. The drug had been added to the reaction mixture in 100 molar excess versus MAb protein and the resultant molar incorporation ratios average 12 (min 8-max 18, n=10 rxns). The conjugation yield and MIR is improved by bringing the final pH of the reaction to 8.0-8.5 before the glutaraldehyde addition. MIR with glutaraldehyde alone average 7-10 while the first linker (the water-soluble carbodiimide, EDCI) contributes 2-4 additional Dox molecules to the protein, presumably via a peptide bond.

This "two-step" procedure (i.e. carbodiimide at a pH of about 5.5-6.0 and glutaraldehyde at a pH of about 8.0-8.5) may be used to link the drugs to proteins other than antibodies. Such proteins include other targeting proteins or polymeric intermediates (e.g., human serum albumin).

Radiolabeling is conducted using any suitable procedure, such as one of the procedures described in Examples 4 or 7 above.

One conjugate prepared by the above-described procedures is doxorubicin-antibody-$^{125}$I, wherein the antibody is a F(ab)'2 fragment of monoclonal antibody NR-ML-05. The procedures described in European Patent Application Publication No. 203,764 (and the p-iodo-phenyl reagents comprising a protein conjugation group, described therein) were used to radioiodinate the conjugate. The chemo-radio-immuno-conjugate was found to effectively localize on melanoma target cells in biodistribution studies using nude mice bearing transplanted tumors. The conjugate effectively eradicated melanoma cells in in vitro cytotoxicity assays (e.g. MTT assays).

The features disclosed in the foregoing description and in the claims may, both separately and in any combination thereof, be material for realising the invention in diverse forms thereof.

## Claims

1. A conjugate comprising an antibody, at least one therapeutic agent, and at least one radionuclide.

2. The conjugate of claim 1 wherein said conjugate comprises a chemotherapeutic agent.

3. The conjugate of claim 1 wherein said chemotherapeutic agent is selected from the group consisting of anti-tumor antibiotics, porphyrin and porphyrin derivatives, anti-tumor alkylating agents, vinca alkaloids, nucleoside analogs, and folic acid analogs.

4. The conjugate of claim 3 wherein said chemotherapeutic agent is selected from the group consisting of doxorubicin, daunorubicin, other anthracycline derivatives, methotrexate, aminopterin, vinblastine, vindesine, bleomycin, hematoporphyrin derivative, dihematoporphyrin ether, mithramycin, mitomycin, chlorambucil, cytosine arabinoside, and fluorodeoxyuridine.

5. The conjugate of claim 1 wherein said conjugate comprises at least one therapeutically effective radionuclide selected from the group consisting of alpha-emitters and beta-emitters.

6. The conjugate of claim 1 wherein said conjugate comprises a chemotherapeutic agent and at least one therapeutically effective radionuclide selected from the group consisting of alpha-emitters and beta-emitters.

7. The conjugate of claim 1 wherein said conjugate comprises a toxin or therapeutically effective fragment thereof.

8. The conjugate of claim 1 wherein said conjugate comprises a toxin or therapeutically effective fragment thereof and a chemotherapeutic agent.

9. The conjugate of claim 1 wherein said conjugate comprises a toxin or a therapeutically effective fragment thereof and a therapeutically effective radionuclide selected from the group consisting of alpha-emitters and beta-emitters.

10. The conjugate of claim 9 wherein said conjugate additionally comprises a chemotherapeutic agent.

11. The conjugate of claims 7, 8, 9, or 10 wherein the toxin is selected from the group consisting of ricin, abrin, diptheria toxin, Pseudomonas exotoxin A, ribosomal inactivating proteins, mycotoxins, trichothecenes, and therapeutically effective fragments thereof.

12. The conjugate of claim 1 or 2 wherein said conjugate comprises a biologic response modifier or therapeutically effective fragment thereof.

13. The conjugate of claim 1 wherein said conjugate comprises a therapeutically effective radionuclide and a biologic response modifier or therapeutically effective fragment thereof.

14. The conjugate of claim 1, 2, 5, 6, 7, 8, 9, 10, or 13 wherein said conjugate additionally comprises a diagnostically effective radionuclide.

15. The conjugate of claim 14 wherein the diagnostically effective radionuclide is selected from the group consisting of $^{99m}$Tc, $^{123}$I, and $^{111}$In.

16. The conjugate of claim 12 wherein said conjugate additionally comprises a diagnostically effective radionuclide.

17. The conjugate of claim 5, 6, 9, 10, or 13 wherein the therapeutically effective radionuclide is selected from the group consisting of $^{125}$I, $^{131}$I, $^{90}$Y, $^{67}$Cu, $^{188}$Re, $^{186}$Re, $^{212}$Bi, and $^{211}$At.

18. The conjugate of claim 1 wherein a plurality of at least one of the therapeutic agents or radionuclides is attached to a polymeric intermediate which is attached to the antibody.

19. The conjugate of claim 1, 2, 5, or 7 wherein the antibody is a monoclonal antibody or a monoclonal antibody fragment.

20. The conjugate of claim 19 wherein the monoclonal antibody or monoclonal antibody fragment is specific for cancer cells.

21. A composition of matter comprising a conjugate of a chemotherapeutic agent and an antibody, said conjugate having an effective amount of a therapeutic radionuclide bound thereto.

22. The composition of claim 21 wherein said chemotherapeutic agent is selected from the group consisting of anti-tumor antibiotics, porphyrin and porphyrin derivatives, anti-tumor alkylating agents, vinca alkaloids, nucleoside analogs, and folic acid analogs.

23. The composition of claim 21 wherein said radionuclide is selected from the group consisting of alpha-emitters and beta-emitters.

24. The composition of claim 22 wherein said chemotherapeutic agent is selected from the group consisting of methotrexate, aminopterin, vinblastine, vindesine, bleomycin, a hematoporphyrin, derivative, dihematoporphyrin ether, mithramycin, mitomycin, chlorambucil, an anthracycline derivative, doxorubicin, daunorubicin, cytosine arabinoside, and fluorodeoxyuridine.

25. The composition of claim 23 wherein said radionuclide is selected from the group consisting of $^{125}I$, $^{131}I$, $^{90}Y$, $^{67}Cu$, $^{188}Re$, $^{186}Re$, $^{211}At$, and $^{212}Bi$.

26. The composition of claim 21, 23, or 25 wherein the radionuclide is in the form of a chelate.

27. The composition of claim 21 or 22 wherein said antibody is a monoclonal antibody or a monoclonal antibody fragment.

28. The composition of claim 27 wherein the monoclonal antibody or monoclonal antibody fragment is specific for cancer cells.

29. A conjugate comprising an antibody, doxorubicin, and at least one radionuclide selected from the group consisting of $^{125}I$, $^{123}I$, $^{131}I$, $^{111}In$, $^{99m}Tc$, $^{186}Re$, $^{188}Re$, $^{211}At$, $^{90}Y$, $^{67}Cu$, and $^{212}Bi$.

30. The conjugate of claim 29 comprising doxorubicin and a radionuclide selected from the group consisting of $^{125}I$, $^{123}I$, and $^{131}I$, attached to a monoclonal antibody.

31. A conjugate comprising a radionuclide selected from $^{111}In$ or $^{90}Y$ attached to hematoporphyrin derivative which is attached to a monoclonal antibody.

32. A conjugate comprising $^{90}Y$ attached to bleomycin which is attached to a monoclonal antibody.

33. A composition comprising a conjugate of at least one therapeutic agent, at least one therapeutically effective radionuclide, and an antibody that binds to the target cells, for use in eradicating target cells within a human or mammalian host.

34. The composition of claim 33 wherein the conjugate comprises a chemotherapeutic agent.

35. A method of preventing target cell proliferation comprising the step of exposing said target cell to an effective amount of a conjugate of an antibody, a chemotherapeutic agent and a radionuclide.

36. The method of claim 34 or 35 wherein said chemotherapeutic agent is selected from the group consisting of anti-tumor antibiotics, porphyrin and porphyrin derivatives, anti-tumor alkylating agents, vinca alkaloids and folic acid analogs.

37. The method of claim 33 or 35 wherein said radionuclide is selected from the group consisting of alpha-emitters and beta-emitters.

38. The method of claim 33 or 35 wherein the target cells are cancer cells and the antibody is a monoclonal antibody or a fragment thereof that binds to the cancer cells.

39. A method of making a conjugate comprising the steps of binding a chemotherapeutic agent to an antibody and binding a radionuclide to one of said chemotherapeutic agent and said antibody.

40. The method of claim 39 wherein a plurality of chemotherapeutic agents are bound to said antibody.

41. The method of claim 40 wherein a plurality of chemotherapeutic agents are bound to a polymeric intermediate which is attached to the antibody.

42. The method of claim 33, 35, or 39 wherein a plurality of radionuclides are bound to a polymeric intermediate which is attached to the antibody.

43. A method of monitoring biodistribution of a therapeutically effective conjugate in vivo, comprising the steps of:

a) attaching a diagnostically effective radionuclide to a conjugate comprising an antibody, a therapeutic agent, and a therapeutically effective radionuclide;

b) administering the resulting conjugate comprising the diagnostically effective radionuclide to a human or mammalian host; and

c) detecting the biodistribution of the diagnostically effective radionuclide within the host.

44. A method for attaching an agent to a protein, comprising the steps of:

a) reacting the protein with a molar excess of the agent under a first set of reaction conditions such that the agent reacts with a first functional group on the protein to bind a first portion of the agent molecules to the protein; and

b) changing the reaction conditions such that unbound agent molecules react with a second functional group on the protein, thereby binding a second portion of the agent molecules to the protein.